(19) Europäisches Patentamt
European Patent Office
Office européen des brevets

(11) Veröffentlichungsnummer: **0 197 407**
**B1**

# (12) EUROPÄISCHE PATENTSCHRIFT

(45) Veröffentlichungstag der Patentschrift: **11.10.89**

(51) Int. Cl.$^4$: **C 07 D339/04**, C 07 C153/09, C 07 C149/20

(21) Anmeldenummer: **86103974.1**

(22) Anmeldetag: **22.03.86**

## (54) Verfahren zur Herstellung von 1,2-Dithiolan-3-pentansäure (Thioctsäure).

(30) Priorität: **11.04.85 DE 3512911**

(43) Veröffentlichungstag der Anmeldung: **15.10.86 Patentblatt 86/42**

(45) Bekanntmachung des Hinweises auf die Patenterteilung: **11.10.89 Patentblatt 89/41**

(84) Benannte Vertragsstaaten: **AT BE CH DE FR GB IT LI LU NL SE**

(56) Entgegenhaltungen:
**US-A- 2 752 373**
**US-A- 2 828 321**
**US-A- 2 980 716**
**Houben-Weyl, Band E 11 (1985), Seiten 135-138**
**Comprehensive Heterocyclic Chemistry, Band 6 (1084), Seiten 801, 802**

(73) Patentinhaber: **ASTA Pharma Aktiengesellschaft**
**Weismüllerstrasse 45**
**D-6000 Frankfurt am Main 1 (DE)**

(72) Erfinder: **Giray, Guenes**
**Goethe Strasse 71**
**D-8752 Kleinostheim (DE)**
Erfinder: **Huthmacher, Klaus, Dr.**
**Lärchenweg 18**
**D-6460 Gelnhausen (DE)**
Erfinder: **Kleemann, Axel, Dr.**
**Bornweg 36**
**D-6052 Mühlheim (DE)**
Erfinder: **Lied, Thomas, Dr.**
**Scheffelstrasse 24**
**D-7753 Allensbach (DE)**

EP 0 197 407 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein neues verbessertes Verfahren zur Herstellung von 1,2-Dithiolan-3-pentansäure (Thioctsäure, α-Liponsäure). Thioctsäure wurde 1953 von den amerikanischen Biochemikern L. J. Reed und J. C. Gunsalus zum erstenmal in kristalliner Form aus Leberextrakten dargestellt. Die optisch aktive (+)-α-Liponsäure ist ein Naturstoff, der in geringer Konzentration im tierischen wie auch im menschlichen Organismus vorkommt. Liponsäure wirkt als eines von mehreren Coenzymen bei der oxidativen Decarboxylierung von Pyruvat und anderen α-Ketosäuren. Es gehört zu den Substanzen, die die Parenchymschäden der Leber günstig beeinflussen (nekrotrope Stoffe).

D, L-Thioctsäure wird als pharmazeutisches Präparat zur Behandlung von akuten und chronischen Lebererkrankungen sowie Vergiftungen eingesetzt. Bei der Behandlung von Krankheiten, die eine hochdosierte, die Leber stark belastende Chemotherapie erfordern, unterstützt Thioctsäure die Regeneration der Leber. Ein weiteres Einsatzgebiet für Thioctsäure ist die Behandlung von Neuropathien.

Zur Darstellung von Thioctsäure sind mehrere vielstufige Synthesen bekannt, die z. B. in den US-Patentschriften 2 752 373, 2 752 374, 2 792 406, 2 980 716, 3 049 549 und 3 223 712 beschrieben sind. Die meisten der bekannten Thioctsäuresynthesen basieren auf der Umsetzung des 5-Chlorcarbonylvaleriansäuremethylester (1) mit Ethylen (2) in Gegenwart von Aluminiumchlorid. Der hierbei gebildete 8-Chlor-6-oxooctansäuremethylester (3) wird dann auf verschiedenen Wegen in Thioctsäure überführt.

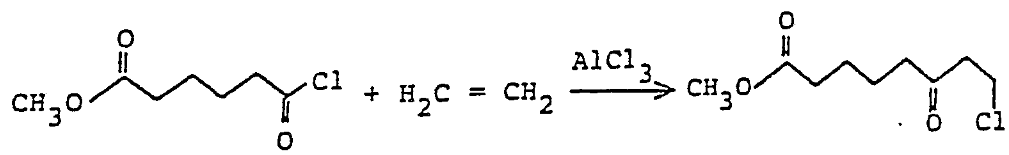


(1) (2) (3)

Die von L. J. Reed und C.-J. Niu in J. Am. Chem. Soc. 77, Seite 416 (1955) beschriebenen Synthesen führen ausgehend von der Zwischenstufe (3) unter Verwendung des sehr aggressiven Phosphortibromids mit einer bescheidenen Gesamtausbeute von 17 % bezogen auf das Säurechlorid (1) zur Thioctsäure. Ausgehend von dem Säurechlorid (1) wird nach dem US-Patent 2 980 716 von L. J. Reed durch ein mehrstufiges Verfahren eine bessere Gesamtausbeute erreicht, allerdings ist die technische Realisierbarkeit dieser Synthese durch eine große Zahl notwendiger Vakuumdestillationen eingeschränkt. Eine weitere Synthese von Thioctsäure beschreiben M. W. Bullock et al., J. Am. Chem. Soc. 79, 1978 (1957) ausgehend von dem 8-Chlor-6-oxooctansäuremethylester (3) über die Einführung der Schwefelatome in das Molekül mittels einem speziellen Cobaltpolysulfidkatalysator und Schwefelwasserstoff. Dieser Syntheseweg ist wirtschaftlich nicht interessant, da der Katalysator nicht in vollem Umfang regeneriert werden kann und zudem bei recht hohen Drucken gearbeitet werden muß.

Nach dem US-Patent 2 828 321 von M. W. Bullock können die entsprechenden 8-Thiosubstituierten-6-oxooctansäurealkylester auch nach folgendem Reaktionsweg hergestellt werden

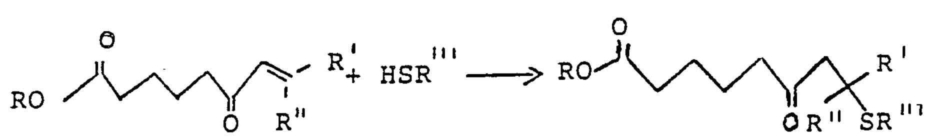

Die auf diese Weise hergestellten Zwischenprodukte zeigen jedoch unter anderem den Nachteil, daß zur Herstellung von Thioctsäure ein zusätzlicher Schritt zur Verseifung des Esters vorgenommen werden muß.

In der US-Patentschrift 2 792 406 von D. S. Acker wird die Darstellung von Thioctsäure durch Umsetzung des 6,8-Dichloroctansäureesters mit Natriumsulfid und Schwefel beschrieben. Hierbei wird zunächst mit dem aus Natriumsulfid und Schwefel sich bildenden Dinatriumdisulfid Chlor gegen Schwefel substituiert, anschließend der Ester verseift und dann angesäuert. Ein entscheidender Nachteil dieses Verfahrens ist die Bildung von polymerer Thioctsäure, die sich nur schwer und unvollständig abtrennen läßt, so daß sich nur in geringer Ausbeute eine reine Thioctsäure gewinnen läßt.

Trotz zahlreicher bekannter Thioctsäure-Synthesen besteht ein Bedarf nach einer wirtschaftlichen Synthese mit einer hohen Ausbeute. Die vorliegende Erfindung betrifft ein Verfahren zur Herstellung von Thioctsäure mit leicht zugänglichen Ausgangsmaterialien, die in einfachen chemischen Operationen ohne drastische Bedingungen umgesetzt werden. Die Synthese weist mit einer Ausbeute von 47 % der Theorie über 8 Stufen eine sehr gute Ausbeute auf.

Das neue Verfahren zur Herstellung von Thioctsäure wird wie folgt beschrieben, wobei R in den angegebenen Formeln stets $C_1$-$C_4$-Alkyl, Phenyl oder Benzyl bedeutet:

1. Umsetzung eines Alkylmercaptans mit Acrylsäurealkylester, in an sich bekannter Weise, zu einem 3-Alkylthiopropionsäurealkylester

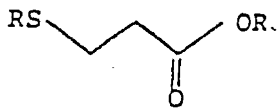

(I)

2. Umsetzung eines 3-Alkylthiopropionsäurealkylesters I mit Alkalihydroxid in an sich bekannter Weise zur 3-Alkylthiopropionsäure

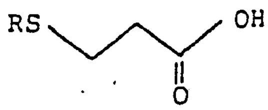

(II)

3. Umsetzung von 3-Alkylthiopropionsäure II mit einem Chlorierungsmittel, wie z. B. Thionylchlorid, Phosphortrichlorid, Phosphorpentachlorid in an sich bekannter Weise zu 3-Alkylthiopropionsäurechlorid

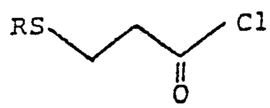

(III)

4. Herstellung eines Enamins aus Cyclopentanon mit einem sekundären Amin, wie z. B. Morpholin, Piperidin, Pyrrolidin, Oxazolidin, N-Methylpiperazin in an sich bekannter Weise zu einem 1-(Dialkylamino)-cyclopenten-1-IV, wobei $R_1$ und $R_2$ einen Ring bedeutet mit $-CH_2-CH_2-O-CH_2-CH_2$ oder $-(CH_2)_5$, $-(CH_2)_4-$, $-CH_2-CH_2-OCH_2-$, $-CH_2-CH_2-N(CH_3)-CH_2-CH_2-$

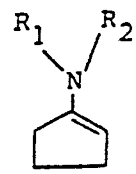

(IV)

5. Umsetzung von 3-Alkylthiopropionsäurechlorid III mit einem Enamin den Cyclopentanons IV in an sich bekannter Weise zu 2-(3-Alkylthiopropionyl)-cyclopentan-1-on

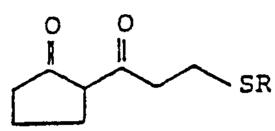

(V)

6. Umsetzung eines 2-(3-Alkylthiopropionyl)-cyclopentan-1-ons V mit einer äquimolaren oder überschüssigen Menge eines Alkalihydroxids, wie z. B. Natriumhydroxid oder Kaliumhydroxid in Wasser, bei einer Temperatur von etwa 20 °C bis etwa 90 °C, vorzugsweise von 50 °C bis 80 °C, insbesondere bei 70 °C bis 75 °C und anschließendem Ansäuern mit einer üblichen Säure, wie z. B. Salzsäure, bis ein neutraler oder leicht saurer pH-Wert vorliegt zur 8-Alkylthio-6-oxooctansäure

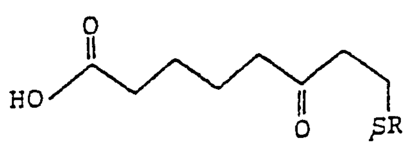

(VI)

7. Umsetzung einer 8-Alkylthio-6-oxooctansäure VI mit einem 4- bis 6-fachen molaren Überschuß eines $C_1$-$C_4$-Alkylmercaptans in Gegenwart einer Halogenwasserstoffsäure, wie z. B. Chlorwasserstoff oder Bromwasserstoff und einer geringen Menge eines sauren anorganischen Salzes, wie z. B. Zinkchlorid, Zinkbromid, Bortrichlorid, Bortrifluorid mit oder ohne Lösungsmittel bei einer Temperatur zwischen —20 °C und 0 °C, vorzugsweise zwischen —10 °C und 0 °C, insbesondere bei —5 °C und 0 °C zu der 6,6,8-Trialkylthiooctansäure

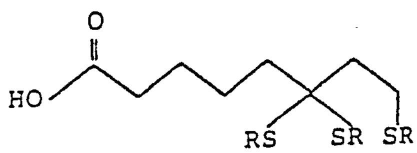

(VII)

8. Umsetzung einer 6,6,8-Trialkylthiooctansäure VII mit einem 6- bis 10-fachen molaren Überschuß Natrium in flüssigem Ammoniak und einem inerten organischen Lösungsmittel, wie z. B. aliphatische symmetrische oder asymmetrische Ether mit $C_1$-$C_5$ Alkylresten, sowie cyclische Ether, insbesondere Dimethylether, Di-n-butylether bei Temperaturen zwischen — 60 °C und — 10 °C, vorzugsweise zwischen — 50 °C und — 35 °C, insbesondere bei — 45 °C und — 35 °C zur 6,8-Dimercaptooctansäure

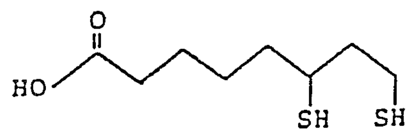

(VIII)

9. Umsetzung der 6,8-Dimercaptooctansäure VIII mit einer in etwa äquimolaren Menge Alkalihydroxid, wie zum Beispiel Natriumhydroxid oder Kaliumhydroxid in Wasser bei einem pH-Wert von ca. 9 mit einem Eisen-(III)-salz, wie zum Beispiel Eisen-(III)-sulfat und Sauerstoff nach einer an sich bekannten Methode (J. Org. Chem. Vol. 43, 3606, 1978) zu der 1,2-Dithiolan-3-pentansäure (Thioctsäure).

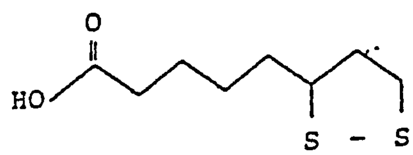

(IX)

Das neue Herstellungsverfahren führt zu der racemischen Form der Thioctsäure IX. Aus der racemischen D, L-Thioctsäure läßt sich in an sich bekannter Weise durch eine Racematspaltung mittels fraktionierter Kristallisation eine optisch aktive, reine enantiomere Form der Thioctsäure gewinnen.

Herstellung von 3-Methylthiopropionsäureethylester (1)

245 g (5,1 Mol) Methylmercaptan werden bei 0 °C in 750 ml Aceton eingeleitet, 2,5 g Triton B zugesetzt und langsam bei 0 °C 500 g (5,8 Mol) Acrylsäuremethylester zugetropft. Man läßt innerhalb 10 Stunden auf Raumtemperatur erwärmen und destilliert überschüssigen Acrylsäuremethylester und Aceton ab. Der Rückstand wird fraktioniert. Man erhält 645 g (95 % der Theorie) 3-Methylthiopropionsäuremethylester vom Siedepunkt 75 °C bei 17 mbar.

Herstellung von 3-Methylthiopropionsäure (II)

In eine Lösung von 240,0 g (6 Mol) Natriumhydroxid in 960 ml Wasser werden 432,0 g (3,22 Mol) 3-Methylthiopropionsäuremethylester eingerührt und anschließend 1,5 Stunden unter Rühren zum Rückfluß erhitzt. Die auf 10 °C abgekühlte Reaktionsmischung wird mit konzentrierter Salzsäure angesäuert (pH 1) und die Phasen getrennt. Nach Extraktion der wäßrigen Phase mit Dichlormethan trocknet man die vereingigten organischen Phasen mit Natriumsulfat. Nach Abdestillieren des Lösungsmittel erhält man 375 g (97 % der Theorie) DC-einheitliche 3-Methylthiopropionsäure, die als Rohprodukt weiterverwendet wird.

Siedepunkt : 127 bis 130 °C bei 14 mbar.

Herstellung von 3-Methylthiopropionsäurechlorid (III)

Zu 952,0 g (8 Mol) Thionylchlorid werden bei Raumtemperatur 745,0 g (6 Mol) 3-Methylthiopropionsäure getropft, wobei sich die Reaktionsmischung abkühlt. Anschließend rührt man 1 Stunde bei 20 °C und 3 Stunden bei 50 °C bis 60 °C. Überschüssiges Thionylchlorid wird bei Normaldruck abdestilliert und hierauf durch fraktionierte Destillation 730,0 g (88 % der Theorie) 3-Methylthiopropionsäurechlorid vom Siedepunkt 73 °C bis 76 °C bei 14 mbar gewonnen.

Herstellung von 1-(Morpholin-4-yl)-cyclopent-1-en (IV)

Die Darstellung erfolgt nach der Vorschrift von S. Hünig und W. Lendle ; Chem. Ber. 93, Seite 909 (1960).

Eine Lösung von 84,0 g (1 Mol) Cyclopentanon und 130,5 g (1,5 Mol) Morpholin in 400 ml Toluol wird 4 Stunden am Wasserabscheider zum Rückfluß erhitzt. Danach wird Morpholin und Toluol abdestilliert und der Rückstand im Vakuum fraktioniert. Man erhält 135 g (88 % der Theorie) 1-(Morpholin-4-yl)-cyclopent-1-en vom Siedepunkt 105 °C bis 109 °C bei 18 mbar.

Herstellung von 2-(3-Methylthiopropionyl)-cyclopentan-1-on V

Zu einer Lösung von 183,6 g (1,2 Mol) 1-(Morpholin-4-yl) cyclopent-1-en und 12?,22 Mol) Triethylamin in 1 200 ml Dichlormethan werden bei 0 °C langsam (2,5 Stunden) 170,0 g (1,23 Mol) 3-Methylthiopropionsäurechlorid in 100 ml Dichlormethan getropft. Nach vollständiger Zugabe wird 1 Stunde bei + 10 °C und 2 Stunden bei + 20 °C nachgerührt und das ausgefallene Triethylaminhydrochlorid abfiltriert. Das Filtrat wird mit 130 ml konzentrierter Salzsäure, sowie 380 ml Wasser versetzt und 10 Stunden bei 30 °C kräftig gerührt. Danach trennt man die Phasen und wäscht die organische Phase mit Wasser und gesättigter Natriumhydrogencarbonatlösung. Nach Trocknung mit Magnesiumsulfat und Abstillieren des Lösungsmittels erhält man 220,0 g (98 % der Theorie) rohes 2-(3-Methylthiopropionyl)-cyclopentan-1-on, das in der nächsten Stufe eingesetzt werden kann. Durch fraktionierende Vakuumdestillation des Rohprodukts werden 166,1 g (74 % der Theorie) reines Diketon als leicht gelbes Öl vom Siedepunkt 120 °C bis 135 °C bei 0,5 mbar erhalten.

Herstellung von 8-Methylthio-6-oxooctansäure (VI)

In eine Lösung von 88,0 g (2,2 Mol) Natriumhydroxid in 1 600 ml Wasser werden 387,0 g (2,08 Mol) rohes 2-(3-Methylthiopropionyl)-cyclopentan-1-on eingerührt und 3 Stunden auf 70 °C bis 75 °C erhitzt. Die auf + 5 °C abgekühlte Reaktionsmischung wird mit konzentrierter Salzsäure angesäuert, die organische Phase abgetrennt und die wäßrige Phase mit Dichlormethan extrahiert. Die vereinigten organischen Phasen werden mit Natriumsulfat getrocknet und im Vakuum eingeengt. Man erhält 415 g (98 % der Theorie) eines semikristallinen Rohprodukts, das nach Umkristallisieren aus Diethylether 280 g (69 % der Theorie) reine, kristalline 8-Methylthio-6-oxooctansäure vom Schmelzpunkt 50 °C bis 52 °C ergibt.

$^1$H-NMR 60 MHz ($CDCl_3$) :

δ = 1,45-1,95 (m, 4H) ;
2,15 (s, 3H) ;
2,25-2,8 (m, 4H) ;
2,82 (s, 4H) ;
10,03 ppm (s, 1H).

IR (KBr) : $\sqrt{}$= 2 500-3 500 (m), 2 935 (m), 1 695 $cm^{-1}$(s).

$C_9H_{16}O_3S$

| | %C | %H | %S |
|---|---|---|---|
| Berechnet: | 52,94 | 7,84 | 15,69 |
| Gefunden: | 53,21 | 8,02 | 14,96 |

Herstellung von 6,6,8-Trimethylthiooctansäure (VII)

Zu einer Mischung von 129,97 g (2,70 Mol) Methylmercaptan und 127,0 g (0,62 Mol) 8-Methylthio-6-oxooctansäure gibt man bei — 10 °C 2,5 g (0,02 Mol) Zinkchlorid und leitet 2 Stunden einen schwachen Strom von trockenem Chlorwasserstoff ein. Die Temperatur der Reaktionsmischung wird dabei durch Kühlen auf 0 °C bis + 5 °C gehalten. Nach beendeter Chlorwasserstoffeinleitung wird 2 Stunden bei 0 °C gerührt und 10 Stunden bei 0 °C bis — 5 °C stehen gelassen. Überschüssiges Methylmercaptan wird abdestilliert, der Rückstand in 200 ml Wasser aufgenommen und nach 0,5 Stunden zweimal mit je 200 ml Dichlormethan extrahiert. Die vereinigten Extrakte werden mit 100 ml Wasser gewaschen, mit Natriumsulfat getrocknet und das Lösungsmittel abdestilliert. Man erhält 173,0 g (98 % der Theorie) rohe 6,6,8-Trimethylthiooctsäure als gelbes Öl.

$^1$H-NMR 60 MHz ($CDCl_3$) :

δ = 1,3-2,1 (m, 8H) ;
2,03 (s, 6H) ;
2,11 (s, 3H) ;
2,15-3,85 (m, 4H) ;
11,0 ppm (s, 1H).

IR (Film) : $\sqrt{}$= 3 000-3 450 (b, s,), 2 920 (s), 2 860 (s), 2 450-2 750 (b, m), 1 700 $cm^{-1}$ (s).

| | %C | %H | %S |
|---|---|---|---|
| Berechnet: | 46,77 | 7,85 | 34,05 |
| Gefunden: | 46,77 | 7,85 | 32,66 |

Herstellung von 6,8-Dimercaptooctansäure (VIII)

In 400 ml Ammoniak werden bei —50 °C 26,0 g (0,092 Mol) 6,6,8-Trimethylthiooctansäure in 150 ml Diethylether eingetragen (0,3 Stunden), die Mischung auf —40 °C erwärmt und 18,0 g (0,780 Mol) Natrium in kleinen Portionen zugegeben. Man läßt 2 Stunden rühren, kühlt wieder auf —50 °C ab, setzt überschüssiges Natrium durch Zugabe von festem Ammoniumchlorid um und destilliert den Ammoniak ab. Der Rückstand wird in 200 ml Wasser aufgenommen und langsam mit 10 %iger Salzsäure angesäuert. Das dabei entstehende Methylmercaptan wird mit einem Stickstoffstrom ausgetragen und in einer Kühlfalle kondensiert. Die wäßrige Lösung extrahiert man zweimal mit je 150 ml Diethylether, trocknet die vereinigten Extrakte mit Natriumsulfat und entfernt das Lösungsmittel im Vakuum. Man erhält 18,3 g (96 % der Theorie) 6,8-Dimercaptooctansäure als hellgelbes Öl.

Herstellung von D, L-Thioctsäure (IX)

Zu 1,54 g (0,038 Mol) Natriumhydroxid in 90 ml Wasser werden 8,0 g (0,038 Mol) 6,8-Dimercaptooctansäure gegeben und die entstehende Lösung mit verdünnter Natronlauge auf pH 9 eingestellt. Man extrahiert zweimal mit je 30 ml Methyltertiärbutylether, trennt die wäßrige Lösung ab und entfernt Etherreste im Vakuum. Die Lösung wird mit 200 ml Wasser verdünnt, 7 mg Eisen-(III)-sulfat zugegeben und unter Rühren bei Raumtemperatur Sauerstoff eingeleitet. Nach 2,5 Stunden hat die Reaktionslösung pH 12,4 erreicht, die Sauerstoffzufuhr wird beendet und das ausgefallene Eisensalz abfiltriert. Bei 5 °C bis 10 °C wird die klar-gelbe Lösung mit 10 %iger Salzsäure angesäuert, 1 Stunde bei pH 1 nachgerührt und das ausgefallene Rohprodukt abfiltriert. Der Niederschlag wird mit Wasser gewaschen, im Vakuum getrocknet und aus Essigsäureethylester/Hexan umkristallisiert. Nach Trocknung erhält man 5,9 g (75 % der Theorie) 1,2-Dithiolan-3-pentansäure als gelbe Kristalle vom Schmelzpunkt 61 °C bis 62 °C.

$^1$H-NMR 60 MHz ($CDCl_3$) :

δ = 1,3-2,30 (m, 8H) ;
2,15-2,80 (m, 2H) ;
3,18 (t, J = 7Hz, 2H) ;
3,3-3,85 (m, 1H) ;
11,3 ppm (s, 1H).

IR (KBr) : $\sqrt{}$= 3 000-3 250 (b, m), 2 935 (s), 2 865 (m), 2 250-2 800 (b, m), 1 690 $cm^{-1}$ (s).

| | %C | %H | %S |
|---|---|---|---|
| Berechnet: | 46,57 | 6,84 | 31,08 |
| Gefunden: | 46,46 | 6,70 | 30,77 |

**Patentansprüche**

1. Verfahren zur Herstellung von 1,2-Dithiolan-3-pentansäure (Thioctsäure) der Formel

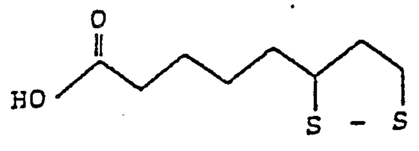

in einer mehrstufigen Reaktion, wobei man in einer letzten Reaktionsstufe 6,8-Dimercaptooctansäure in alkalischer Lösung mit einem Eisen-(III)-salz und Sauerstoff oxidiert, dadurch gekennzeichnet, daß man in einer ersten Reaktionsstufe

a) 2-(3-Alkylthiopropionyl)-cyclopentan-1-on in alkalisch-wäßriger Lösung bei einer Temperatur von etwa 20 °C bis etwa 50 °C zu der entsprechenden Carbonsäure der Formel VI umsetzt, wobei R jeweils $C_1$-$C_4$-Alkyl, phenyl oder benzyl bedeutet,

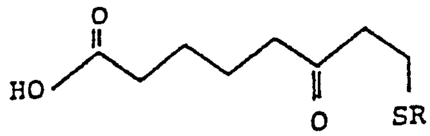

(VI)

und in einer zweiten Reaktionsstufe

b) die erhaltene Carbonsäure VI mit einem Alkylmercaptan bei einer Temperatur zwischen — 20 °C und 0 °C zu dem entsprechenden Thioketal der Formel VII umsetzt

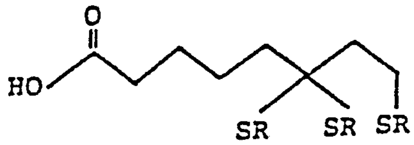

(VII)

und in einer dritten Reaktionsstufe

c) das erhaltene Thioketal der Formel VII mit Natrium in flüssigem Ammoniak bei Temperaturen zwischen — 60 °C und — 10 °C zu 6,8-Dimercaptooctansäure umsetzt.

2. 8-Alkylthio-6-oxooctansäure der Formel VI, wobei R $C_1$-$C_4$-Alkyl bedeutet.

3. 6,6,8-Trialkylthiooctansäure der Formel VII, wobei R $C_1$-$C_4$-Alkyl bedeutet.

## Claims

1. A process for the production of 1,2-dithiolan-3-pentanoic acid (thioctic acid) corresponding to the following formula

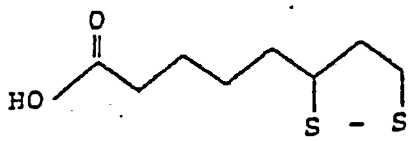

in a multistep reaction, 6,8-dimercaptooc?ancid being oxidized in alkaline solution with an iron (III) salt and oxygen in a final reaction step, characterized in that, in a first reaction step,

a) 2-(3-alkylthiopropionyl)-cyclopentan-1-one is reacted in alkaline aqueous solution at a temperature of from about 20 °C to about 90 °C to form the corresponding carboxylic acid of formula VI where R is $C_{1-4}$ alkyl, phenyl or benzyl

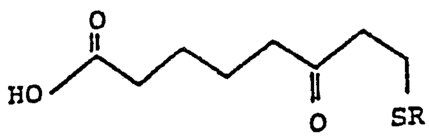

(VI)

and, in a second reaction step,

b) the carboxylic acid VI obtained is reacted with an alkyl mercaptan at a temperature of — 20 °C to 0 °C to form the corresponding thioketal of formula VII

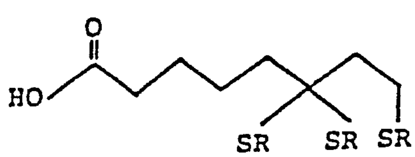

(VII)

and, in a third reaction step,

c) the thioketal VII obtained is reacted with sodium in liquid ammonia at temperatures in the range from — 60 °C to — 10 °C to form 6,8-dimercaptooctanoic acid.

2. 8-Alkylthio-6-oxooctanoic acid corresponding to formula VI in which R is $C_{1-4}$ alkyl.

3. 6,6,8-Trialkylthiooctanoic acid corresponding to formula VII in which R is $C_{1-4}$ alkyl.

**Revendications**

1. Procédé de préparation d'acide 1,2-dithiolane-3-pentanoïque (acide thioctique) de formule

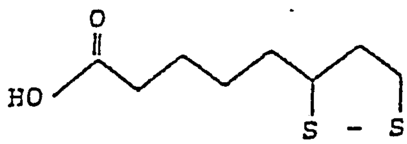

en une réaction en plusieurs étapes dans laquelle, dans une dernière étape de la réaction, on oxyde de l'acide 6,8-dimercapto-octanoïque en solution alcaline avec un sel ferrique et de l'oxygène, caractérisé en ce que, dans une première étape de réaction

a) une 2-(3-alkylthiopropionyl)-cyclopentane-1-one en solution hydro-alcaline est transformée, à une température d'environ 20 °C à environ 90 °C, en l'acide carboxylique de formule VI correspondant, R représentant chaque fois un groupement alkyle en $C_1$-$C_4$, phényle ou benzyle

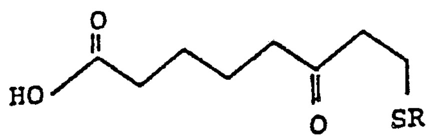

(VI)

puis, dans une deuxième étape de réaction,

b) l'acide carboxylique VI obtenu est mis à réagir avec un alkylmercaptan à une température comprise entre — 20 °C et 0 °C pour l'obtention du thiocétal de formule VII correspondant

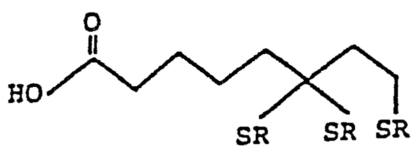

(VII)

et, dans une troisième étape de réaction,

c) le thiocétal de formule VII obtenu est mis à réagir avec du sodium dans l'ammoniac liquide à des températures comprises entre — 60 °C et — 10 °C pour l'obtention d'acide 6,8-dimercapto-octanoïque.

2. Acide 8-alkylthio-6-oxo-octanoïque de formule VI, R représentant un groupement alkyle en $C_1$-$C_4$.

3. Acide 6,6,8-trialkylthio-octanoïque de formule VII, R représentant un groupement alkyle en $C_1$-$C_4$.

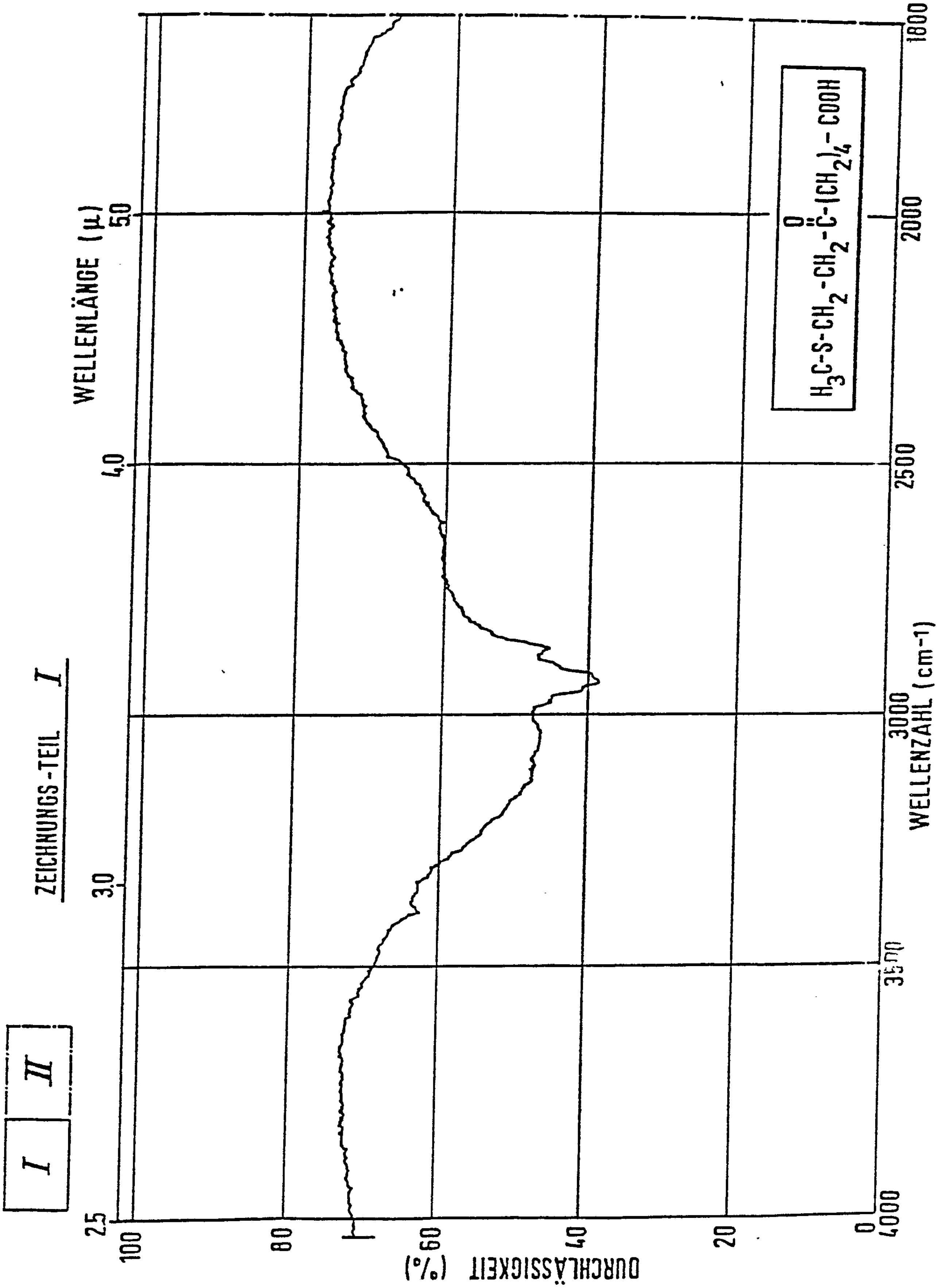
I
II
ZEICHNUNGS-TEIL I
WELLENLÄNGE (μ)
2.5
3.0
4.0
5.0
WELLENZAHL (cm-1)
4000
3500
3000
2500
2000
1800
DURCHLÄSSIGKEIT (%)
100
80
60
40
20
0
$H_3C-S-CH_2-CH_2-\overset{O}{\overset{\|}{C}}-(CH_2)_4-COOH$

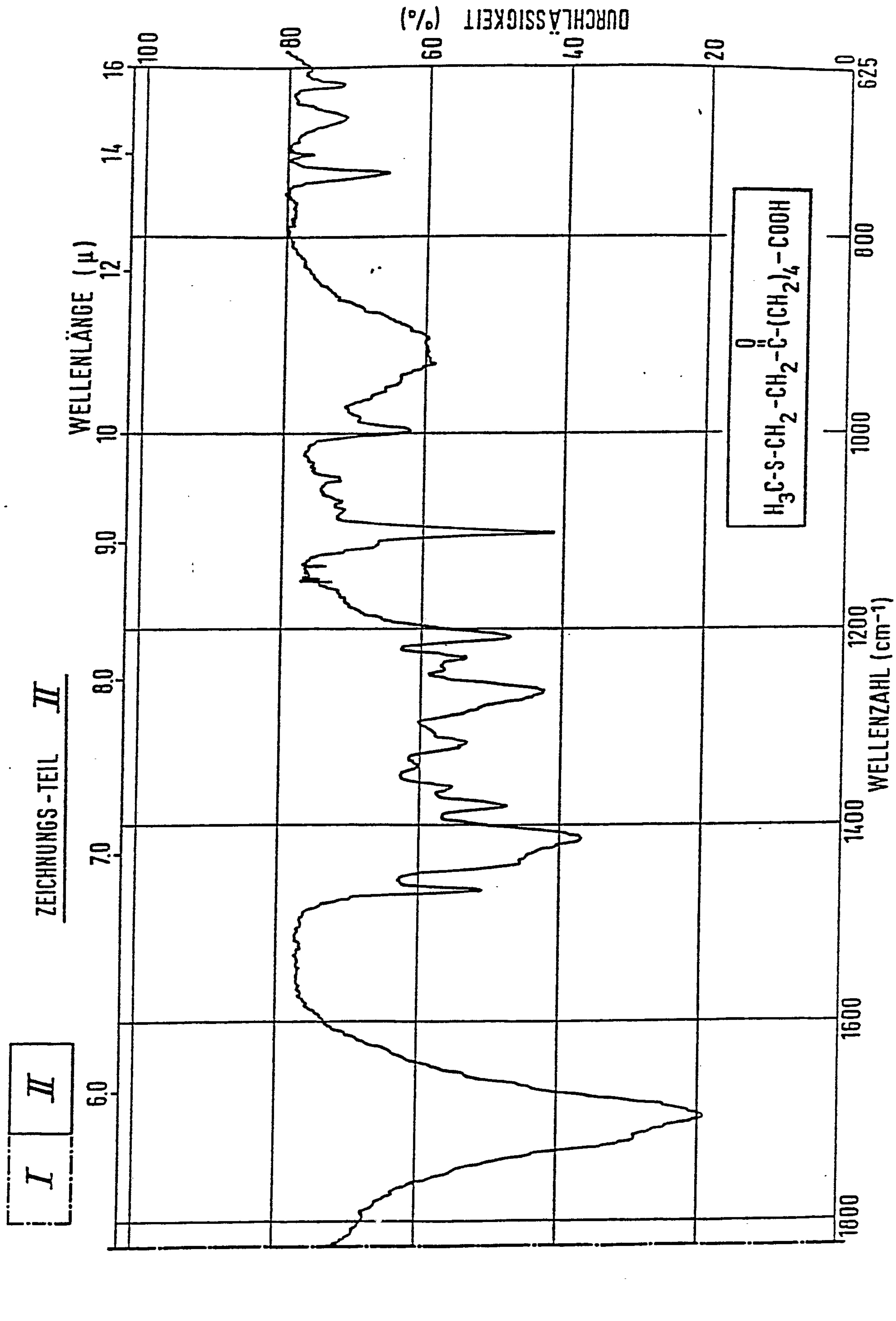
DURCHLÄSSIGKEIT (%)
100
80
60
40
20
0
WELLENLÄNGE (μ)
16
14
12
10
9,0
8,0
7,0
6,0
WELLENZAHL (cm-1)
625
800
1000
1200
1400
1600
1800
ZEICHNUNGS-TEIL II
I
II
H3C-S-CH2-CH2-C(O)-(CH2)4-COOH